# FASCICULE DE BREVET EUROPEEN

(11) **EP 2 455 115 B1**
(45) Date de publication et mention de la délivrance du brevet: **25.06.2014**
(21) Numéro de dépôt: 11188200.7
(22) Date de dépôt: 08.11.2011
(51) Int. Cl.: A61M 1/00, A61B 17/3207, A61B 10/02

(54) **Vecteur de prélèvement pour tissus en particulier adipeux**
Entnahmeträger für Gewebe, insbesondere für adipöses Gewebe
Sampling carrier for tissue, in particular adipose

(30) Priorité: 19.11.2010 FR 1059533
(43) Date de publication de la demande: 23.05.2012
(73) Titulaire: Persat, Jean-Charles, Canton de GENEVE (CH)
(72) Inventeur: Perast, Jean-Charles, Collex-Bossy Canton de Genève (CH)
(74) Mandataire: Thibault, Jean-Marc

(56) Documents cités:
- EP-A1- 0 800 794
- EP-A1- 1 092 515
- EP-A1- 2 140 820
- WO-A1-01/00100
- DE-C1- 10 049 813
- FR-A1- 2 942 410
- US-A- 4 111 207
- US-A- 5 527 332
- US-A1- 2005 090 765

## Description

La présente invention concerne le domaine technique général du prélèvement de tissu adipeux du corps humain à l'aide de vecteurs de prélèvement désignés également sous le terme de canules ou d'aiguilles.

L'objet de l'invention trouve des applications particulièrement avantageuses dans le domaine de certains traitements de thérapie cellulaire ayant recours aux cellules souches du tissu adipeux prélevé, dans le domaine de l'autogreffe de tissus pour la chirurgie réparatrice (sein, visage, main, ...) ou pour la chirurgie reconstructive dans le complément des pertes de substances (os), ou encore dans le domaine de la lipoaspiration de zones adipeuses.

Dans l'état de la technique, il est connu de prélever des tissus adipeux à l'aide d'une canule réalisée sous la forme d'une aiguille ou d'un tube présentant un alésage interne de section circulaire et une face externe de section circulaire. Cette canule comporte généralement une extrémité distale mousse atraumatique destinée à être insérée dans les tissus et une extrémité proximale de liaison à un élément de préhension. Le tube est aménagé pour comporter un ou plusieurs orifices de prélèvement des tissus adipeux, communiquant avec l'alésage interne de la canule qui est généralement relié à une source d'aspiration.

En pratique, le prélèvement de tissus adipeux conduit à des lésions tissulaires ou à des arrachements tissulaires. Un tel prélèvement provoque fréquemment des phénomènes inflammatoires post prélèvement sévères et génère des saignements étendus. Par ailleurs, un tel prélèvement présente un rendement relativement faible avec des tissus prélevés de faible qualité.

Le document US 4 111 207 décrit un vecteur de prélèvement selon le préamble de la revendication 1.

Dans le domaine des cathéters, il est connu par le document DE 100 49 813, un cathéter assurant l'ablation de la valve aortique calcifiée. Ce cathéter est muni, à son extrémité distale, d'un système d'ablation comportant un tube présentant une face interne de section circulaire et une face externe de section circulaire. Le tube comporte un orifice de prélèvement bordé par un bord coupant. A l'intérieur du tube, est monté mobile, un outil de coupe présentant une ouverture délimitant un bord de coupe. Un tel système d'ablation est de conception et de mise en oeuvre complexes en raison notamment de la présence d'un outil de coupe mobile. Par ailleurs, un tel système d'ablation ne permet pas d'obtenir un prélèvement précis et de qualité en raison du principe même du système d'ablation.

La présente invention vise à remédier aux inconvénients de l'art antérieur en proposant un vecteur de prélèvement minimisant l'effet d'arrachement des tissus adipeux, tout en permettant de prélever des ensembles cellulaires calibrés et de qualité.

Un autre objet de l'invention est de proposer un vecteur de prélèvement de tissus adipeux de conception simple, permettant un geste de prélèvement transcutané indolore et rapide.

Pour atteindre un tel objectif, l'objet de l'invention, concerne un vecteur de prélèvement pour tissus réalisé sous la forme d'un tube présentant une face interne de section circulaire et une face externe de section circulaire, le tube comportant une extrémité distale mousse et au moins un orifice de prélèvement s'étendant symétriquement par rapport à un plan transversal et à un plan longitudinal, l'orifice étant bordé en périphérie par deux bords transversaux symétriques par rapport au plan transversal et reliées à deux bords longitudinaux symétriques par rapport au plan longitudinal.

Selon l'invention :
- les bords transversaux possèdent une arête coupante située à l'intersection entre lesdits bords et la face interne, chaque bord transversal formant, avec la face interne, un angle de taillant compris entre 6 et 18°,
- les bords longitudinaux sont aménagés en cuvette par rapport aux bords transversaux, avec un profil selon le plan longitudinal différent du profil des bords transversaux.

De plus, le vecteur selon l'invention peut présenter en outre en combinaison au moins l'une et/ou l'autre des caractéristiques additionnelles suivantes :
- l'arête coupante de chaque bord transversal comporte deux segments convergents reliés entre eux par un segment de liaison présentant un profil différent du profil des segments convergents,
- l'arête coupante de chaque bord transversal comporte deux segments convergents reliés entre eux par un segment de liaison arrondi,
- chaque bord transversal présente dans le plan longitudinal, un profil plan,
- chaque bord transversal présente dans le plan longitudinal, un profil convexe,
- chaque bord transversal est prolongé dans le sens longitudinal et de part et d'autre du plan longitudinal, par une zone de dépouille,
- chaque bord longitudinal présente deux segments courbes convergents et reliés entre eux par un segment droit,
- le profil de chaque bord longitudinal présente deux segments courbes convergents et reliés entre eux par un segment arrondi.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective d'un exemple de réalisation, d'un vecteur de prélèvement conforme à l'invention.
La **Figure 2** est une vue de côté du vecteur de prélèvement illustré à la **Fig. 1.**
La **Figure 3** est une vue de dessus du vecteur de prélèvement illustré aux **Fig. 1** et **2****.**
La **Figure 4** est une vue en coupe élévation prise sensiblement selon les lignes IV-IV de la **Fig. 3****.**
Les **Figures 5A** à **5C** sont des coupes transversales prises respectivement selon les lignes A B C de la **Fig. 4** d'un exemple de réalisation d'une ouverture de prélèvement.
Les **Figures 5D** à **5F** sont des coupes transversales prises respectivement selon les lignes D E F de la **Fig. 4** d'un autre exemple de réalisation d'une ouverture de prélèvement.

Tel que cela ressort plus précisément des Figures, l'objet de l'invention concerne un vecteur de prélèvement **1** tel qu'une canule ou une aiguille au sens général adaptée pour le prélèvement de tissus adipeux dans le corps humain. Cette canule de prélèvement **1** comporte un tube ou une aiguille tubulaire **2** semi rigide présentant un axe longitudinal **X** de symétrie. Avantageusement, ce tube **2** est réalisé en un matériau métallique en acier inoxydable.

Le tube **2** comporte une extrémité distale **3** arrondie ou hémisphérique fermée. Le tube **2** présente ainsi une extrémité **3** dite mousse atraumatique. Le tube **2** comporte à l'opposé de l'extrémité distale **3,** une extrémité proximale **4** de préhension par exemple raccordée à un dispositif de prélèvement non représenté de tout type connu en soi. Par exemple un tel dispositif de prélèvement peut être une seringue ou un système d'aspiration du type source de vide.

Le tube **2** présente un alésage ou un canal interne **6** délimité par la face interne tubulaire **7** présentant une section droite transversale interne de préférence circulaire. Le tube **2** présente également une face externe **8** tubulaire présentant une section droite transversale externe de préférence circulaire. Le tube **2** présente ainsi une paroi d'épaisseur **e** constante.

La canule de prélèvement **1** selon l'invention comporte également au moins une et dans l'exemple illustré deux ouvertures ou orifices de prélèvement **11.** Chaque orifice de prélèvement **11** est débouchant pour assurer une communication entre les faces interne **7** et externe **8** du tube **2.**

Chaque orifice de prélèvement **11** s'étend symétriquement par rapport à un plan transversal **T** et à un plan longitudinal **L.** Le plan transversal **T** est perpendiculaire à l'axe longitudinal **X** et au plan longitudinal **L** passant par l'axe longitudinal **X.**

Chaque orifice de prélèvement **11** est bordé en périphérie par deux bords transversaux **13** qui s'étendent de manière symétrique par rapport au plan transversal **T.** Ces deux bords transversaux **13** sont reliés à deux bords longitudinaux **14** s'étendant de manière symétrique par rapport au plan longitudinal **L.** Chaque bord transversal **13** s'étend de manière symétrique à partir du plan longitudinal **L** en s'éloignant de ce plan longitudinal **L.** Chaque bord longitudinal **14** s'étend de manière symétrique à partir du plan transversal **T** en s'éloignant de ce plan transversal **T** pour venir se raccorder à chaque extrémité, aux bords transversaux **13.** Ainsi, chaque orifice de prélèvement **11** se trouve pourvue, sur toute sa périphérie, de deux bords transversaux **13** et de deux bords longitudinaux **14,** aménagés à partir de la face externe **8** du tube selon des caractéristiques qui vont être décrites dans la suite de la description.

Conformément à l'invention, chaque bord transversal **13** possède une arête coupante **16** située à l'intersection entre le bord transversal **13** et la face interne **7.** Chaque bord transversal **13** forme avec la face interne **7,** un angle de taillant a dont le sommet correspond à l'arête coupante **16.** Avantageusement, l'angle de taillant α possède une valeur comprise entre 6 et 18°. Les deux bords transversaux **13** qui s'étendent tête-bêche l'un en face de l'autre présentent des zones de coupe **C** qui agissent, comme cela sera montré dans la suite de la description, comme des guillotines permettant de sectionner les tissus sans arrachement.

Selon une caractéristique avantageuse de réalisation, l'arête coupante **16** de chaque bord transversal **13** comporte deux segments **16₁** convergents vers le plan longitudinal **L** en direction opposée du plan transversal de symétrie **T** et s'étendent de manière symétrique par rapport au plan longitudinal **L.** Ces deux segments convergents **16₁** sont reliés entre eux par un segment de liaison **16₂** centré selon le plan longitudinal **L.** Ce segment de liaison **16₂** présente un profil différent du profil des segments convergents **16₁.**

Selon une variante avantageuse de réalisation, les deux segments convergents **16₁** sont droits et sont reliés entre eux par un segment de liaison **16₂** de forme arrondie ou demi-circulaire. Il est à noter que compte tenu de la forme circulaire du tube **2,** le profil des bords transversaux **13** et par suite des segments convergents **16₁** et de liaison **16₂** ne s'établit pas selon un même niveau mais évolue selon une partie de la hauteur du tube prise selon le plan longitudinal **L.**

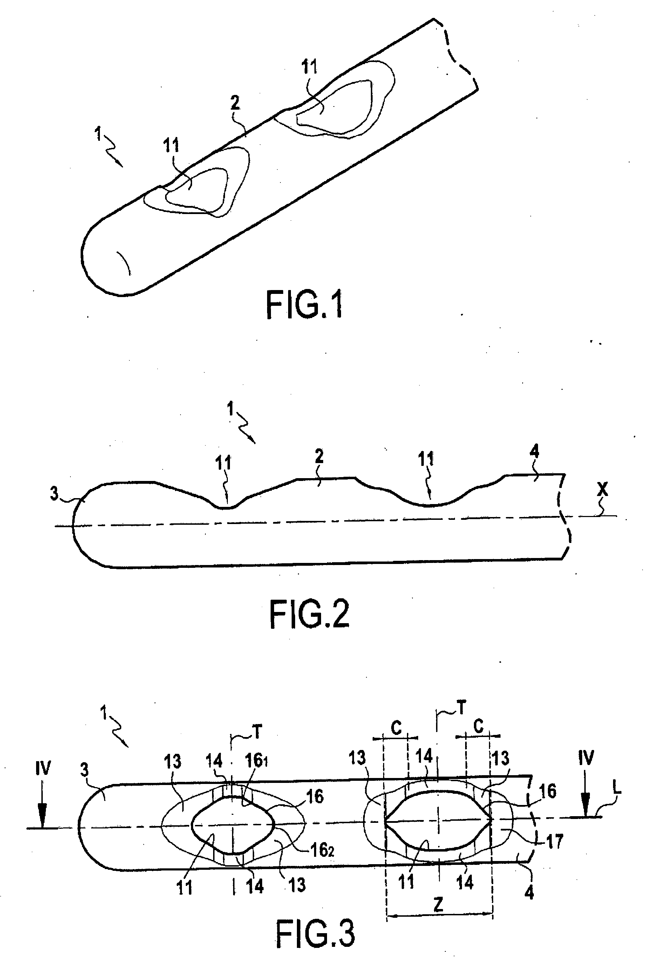

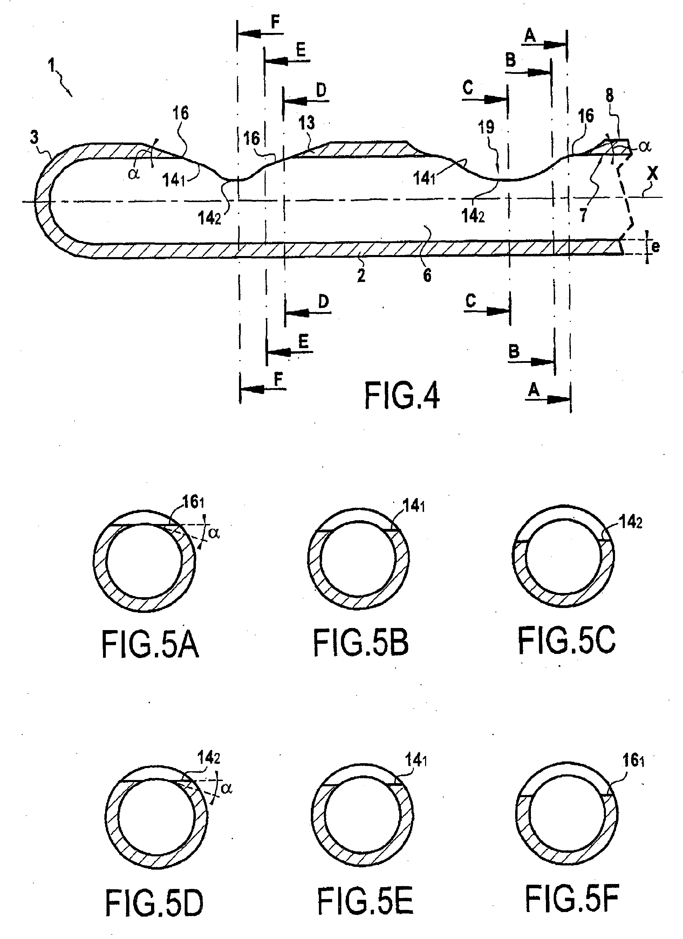

Tel que cela apparaît plus précisément aux **Fig. 2** et **4****,** les bords transversaux **13** peuvent présenter dans le plan longitudinal **L** des profils différents. Sur les dessins, le tube **2** comporte deux orifices de prélèvement **11** avec des profils différents. Ainsi, pour l'orifice de prélèvement **11** situé le plus proche de la partie distale **3,** chaque bord transversal **13** présente dans le plan longitudinal **L,** un profil plan. En ce qui concerne l'orifice de prélèvement **11** situé le plus éloigné de la partie distale **3,** chaque bord transversal **13** présente dans le plan longitudinal **L,** un profil convexe. Avantageusement, chaque bord transversal **13** est prolongé dans le sens longitudinal et de part et d'autre du plan longitudinal **L,** par une zone de dépouille **17** servant de surface d'appui pour le tissu adipeux.

Selon une caractéristique de l'invention, les deux bords longitudinaux **14** sont aménagés en cuvette **19** par rapport aux bords transversaux **13,** avec un profil selon le plan longitudinal **L** qui est différent du profil des bords transversaux **13.** Chaque bord longitudinal **14** possède ainsi une cuvette **19** situé au-delà du bord transversal **13** et par suite de l'arête coupante **16.** En d'autres termes, il apparaît une discontinuité de profil entre les bords transversaux **13** et les bords longitudinaux **14.** Cette discontinuité de profil ou ce « saut de profil » pour les bords périphériques de l'orifice de prélèvement **11** contribue au bon positionnement du tissu adipeux à l'intérieur dudit orifice, comme cela sera montré dans la suite de la description.

Bien entendu, il peut être envisagé divers profils pour les bords longitudinaux **14.** En ce qui concerne l'orifice de prélèvement **11** situé le plus proche de la partie distale **3,** le profil de chaque bord longitudinal **14** présente dans le plan longitudinal **L,** deux segments courbes convergents **14₁** reliés entre eux par un segment droit **14₂** parallèle à l'axe **X.** En ce qui concerne l'orifice de prélèvement **11** situé le plus éloigné de l'extrémité distale **3,** le profil de chaque bord longitudinal **14** présente dans le plan longitudinal **L,** deux segments courbes convergents **14₁** reliés entre eux par un segment arrondi **14₂.**

Il doit être considéré que la cuvette **19** présentée par les bords longitudinaux **14** permet d'assurer le positionnement du tissu adipeux afin qu'il s'étende à l'intérieur du canal **6** en dessous du niveau d'extension des arêtes coupantes **16.** Aussi, un mouvement de translation suivant la direction de l'axe longitudinal **X** conduit à une coupe franche du tissu adipeux, situé dans la zone de piégeage **Z** du tissu adipeux, située entre les deux extrémités de l'orifice **11** prises dans le plan longitudinal **L.** Il est à noter que le tissu adipeux permet d'être également en contact sur la face de dépouille **17** lors de la phase de prélèvement favorisant l'effet de coupe du tissu adipeux.

Il ressort de la description qui précède que la canule de prélèvement **1** permet de prélever un tissu en le sectionnant par l'effet conjugué des deux arrêtes coupantes **16** agissant comme une guillotine sur le tissu adipeux inséré entre ces deux bords transversaux **13.** Le tissu adipeux prélevé est calibré et dépend de la largeur de l'orifice **11,** la longueur de l'orifice **11,** la profondeur de l'orifice **11** prise dans le plan longitudinal **L** et également du profil de l'arête coupante **16.** Dans le cas d'une application de la canule à une technique de lipoaspiration, il peut être envisagé de raccorder le canal **6** à un système d'aspiration de vide contrôlée telle une seringue ou une source de vide.

Le vecteur de prélèvement **1** selon l'invention présente une conception simple tout en étant de mise en oeuvre relativement facile. Le vecteur de prélèvement **1** se présente sous la forme d'un élément tubulaire monobloc sans pièce mobile.

Dans l'exemple illustré, la canule comporte deux orifices de prélèvement **11.** Bien entendu, la canule selon l'invention peut comporter un nombre différent d'orifices répartis selon une génératrice du tube ou selon deux génératrices symétriquement opposées ou distribués sur toute la périphérie du tube en étant décalés selon la longueur du tube pour ne pas fragiliser le tube.

L'invention n'est pas limitée aux exemples décrits et représentés car diverses modifications peuvent y être apportées sans sortir de son cadre.

## Revendications

1. Vecteur de prélèvement pour tissus réalisé sous la forme d'un tube (**2**) présentant une face interne (**7**) de section circulaire et une face externe (**8**) de section circulaire, le tube comportant une extrémité distale (**3**) mousse et au moins un orifice de prélèvement (**11**) s'étendant symétriquement par rapport à un plan transversal (**T**) et à un plan longitudinal (**L**), l'orifice (**11**) étant bordé en périphérie par deux bords transversaux (**13**) symétriques par rapport au plan transversal (**T**) et reliées à deux bords longitudinaux (**14**) symétriques par rapport au plan longitudinal (**L**), **caractérisé en ce que** :
- les bords transversaux (**13**) possèdent une arête coupante (**16**) située à l'intersection entre lesdits bords et la face interne (**7**), chaque bord transversal formant, avec la face interne (**7**), un angle de taillant (α) compris entre 6 et 18°,
- les bords longitudinaux (**14**) sont aménagés en cuvette (**19**) par rapport aux bords transversaux (**13**), avec un profil selon le plan longitudinal différent du profil des bords transversaux (**13**).

2. Vecteur de prélèvement selon la revendication 1, **caractérisé en ce que** l'arête coupante (**16**) de chaque bord transversal (**13**) comporte deux segments convergents (**16₁**) reliés entre eux par un segment de liaison (**16₂**) présentant un profil différent du profil des segments convergents.

3. Vecteur de prélèvement selon la revendication 2, **caractérisé en ce que** l'arête coupante (**16**) de chaque bord transversal (**13**) comporte deux segments convergents reliés entre eux par un segment de liaison arrondi.

4. Vecteur de prélèvement selon l'une des revendications 1 à 3, **caractérisé en ce que** chaque bord transversal (**13**) présente dans le plan longitudinal (**L**), un profil plan.

5. Vecteur de prélèvement selon l'une des revendications 1 à 3, **caractérisé en ce que** chaque bord transversal (**13**) présente dans le plan longitudinal (**L**), un profil convexe.

6. Vecteur de prélèvement selon l'une des revendications 1 à 5, **caractérisé en ce que** chaque bord transversal (**13**) est prolongé dans le sens longitudinal et de part et d'autre du plan longitudinal **(**L), par une zone de dépouille (**17**).

7. Vecteur de prélèvement selon la revendication 1, **caractérisé en ce que** le profil de chaque bord longitudinal (**14**) présente deux segments courbes convergents (**14₁**) et reliés entre eux par un segment droit (**14₂**).

8. Vecteur de prélèvement selon la revendication 1, **caractérisé en ce que** le profil de chaque bord longitudinal (**14**) présente deux segments courbes (**14₁**) convergents et reliés entre eux par un segment arrondi (**14₂**).

## Patentansprüche

1. Entnahmeträger für Gewebe, der in Form einer Röhre (2) ausgebildet ist, die eine Innenseite (7) mit kreisförmigem Querschnitt und eine Außenseite (8) mit kreisförmigem Querschnitt aufweist, wobei die Röhre ein stumpfes distales Ende (3) und wenigstens eine Entnahmeöffnung (11), die sich zu einer Querebene (T) und zu einer Längsebene (L) symmetrisch erstreckt, umfasst, wobei die Öffnung (11) am Umfang durch zwei Querränder (13) eingefasst ist, die zu der Querebene (T) symmetrisch sind und die mit zwei Längsrändern (14), welche zu der Längsebene (L) symmetrisch sind, verbunden sind, **dadurch gekennzeichnet, dass**:
- die Querränder (13) eine Schneidkante (16), die an dem Schnittpunkt zwischen den Rändern und der Innenseite (7) gelegen ist, besitzen, wobei jeder Querrand mit der Innenseite (7) einen Keilwinkel (α) zwischen 6 und 18° bildet,
- die Längsränder (14) gegenüber den Querrändem (13) muldenförmig (19) ausgebildet sind, mit einem Profil entlang der Längsebene, das von dem Profil der Querränder (13) abweicht.

2. Entnahmeträger nach Anspruch 1, **dadurch gekennzeichnet, dass** die Schneidkante (16) eines jeden Querrandes (13) zwei konvergente Segmente (16₁) umfasst, die durch ein Verbindungssegment (16₂), das ein von dem Profil der konvergenten Segmente abweichendes Profil aufweist, untereinander verbunden sind.

3. Entnahmeträger nach Anspruch 2, **dadurch gekennzeichnet, dass** die Schneidkante (16) eines jeden Querrandes (13) zwei konvergente Segmente umfasst, die durch ein abgerundetes Verbindungssegment untereinander verbunden sind.

4. Entnahmeträger nach einem der Ansprüche 1 bis 3, **dadurch gekennzeichnet, dass** jeder Querrand (13) in der Längsebene (L) ein ebenes Profil aufweist.

5. Entnahmeträger nach einem der Ansprüche 1 bis 3, **dadurch gekennzeichnet, dass** jeder Querrand (13) in der Längsebene (L) ein konvexes Profil aufweist.

6. Entnahmeträger nach einem der Ansprüche 1 bis 5, **dadurch gekennzeichnet, dass** jeder Querrand (13) in der Längsrichtung sowie auf beiden Seiten der Längsebene (L) durch einen Freibereich (17) fortgesetzt ist.

7. Entnahmeträger nach Anspruch 1, **dadurch gekennzeichnet, dass** das Profil eines jeden Längsrandes (14) zwei konvergente und durch ein gerades Segment (14₂) untereinander verbundene gekrümmte Segmente (14₁) aufweist.

8. Entnahmeträger nach Anspruch 1, **dadurch gekennzeichnet, dass** das Profil eines jeden Längsrandes (14) zwei konvergente und durch ein abgerundetes Segment (14₂) untereinander verbundene gekrümmte Segmente (14₁) aufweist.

## Claims

1. A tissue-sampling tool made in the form of a tube (2) presenting an inside face (7) of circular section and an outside face (8) of circular section, the tube having a blunt distal end (3) and at least one sampling orifice (11) extending symmetrically relative to a transverse plane (T) and to a longitudinal plane (L), the orifice (11) being peripherally bordered by two transverse borders (13) that are symmetrical relative to the transverse plane (T) and that are connected to two longitudinal borders (14) that are symmetrical relative to the longitudinal plane (L), the tool being **characterized in that**:
· each transverse border (13) possesses a cutting edge (16) situated at the intersection between said borders and the inside face (7), each transverse border co-operating with the inside face (7) to form a cutting angle (α) lying in the range 6° to 18°; and
· the longitudinal borders (14) are arranged to have a cup-shape (19) relative to the transverse borders (13), with a profile in the longitudinal plane that is different from the profile of the transverse borders (13).

2. A sampling tool according to claim 1, **characterized in that** the cutting edge (16) of each transverse border (13) comprises two converging segments (16₁) that are connected together by a connection segment (16₂) presenting a profile that is different from the profile of the converging segments.

3. A sampling tool according to claim 2, **characterized in that** the cutting edge (16) of each transverse border (13) comprises two converging segments connected together by a rounded connection segment.

4. A sampling tool according to any one of claims 1 to 3, **characterized in that** each transverse border (13) presents a plane profile in the longitudinal plane (L).

5. A sampling tool according to any one of claims 1 to 3, **characterized in that** each transverse border (13) presents a convex profile in the longitudinal plane (L).

6. A sampling tool according to any one of claims 1 to 5, **characterized in that** each transverse border (13) is extended in the longitudinal direction and on either side of the longitudinal plane (L) by a tapering zone (17).

7. A sampling tool according to claim 1, **characterized in that** the profile of each longitudinal border (14) presents two converging curved segments (14₁) that are connected together by a straight segment (14₂).

8. A sampling tool according to claim 1, **characterized in that** the profile of each longitudinal border (14) presents two converging curved segments (14₁) that are connected together by a rounded segment (14₂).
